Europäisches Patentamt

European Patent Office

Office européen des brevets

(19)

(11) Numéro de publication : **0 354 142 B1**

(12) **FASCICULE DE BREVET EUROPEEN**

(45) Date de publication du fascicule du brevet :
08.04.92 Bulletin 92/15

(51) Int. Cl.⁵ : **A61F 2/36**

(21) Numéro de dépôt : **89420283.7**

(22) Date de dépôt : **27.07.89**

(54) **Tige pour prothèse totale de hanche.**

(30) Priorité : **28.07.88 FR 8810452**

(43) Date de publication de la demande :
**07.02.90 Bulletin 90/06**

(45) Mention de la délivrance du brevet :
**08.04.92 Bulletin 92/15**

(84) Etats contractants désignés :
**BE DE ES FR GB IT NL**

(56) Documents cités :
**EP-A- 0 135 755**
**EP-A- 0 241 846**
**EP-A- 0 257 222**
**FR-A- 1 481 424**
**FR-A- 2 610 823**
**US-A- 4 714 470**

(73) Titulaire : **Bascoulergue , Gérard**
**45 Boulevard Daloz**
**F-62520 Le Touquet Paris Plage (FR)**
Titulaire : **Basso , Maurice**
**57 Avenue Foch**
**F-43000 Le Puy en Velay (FR)**
Titulaire : **Debiesse, Jean-Louis**
**Chemin de Bel Air**
**F-38200 Vienne (FR)**
Titulaire : **Eyraud, Guy**
**Le Guillonnier Ville sous Anjou**
**F-38150 Roussillon (FR)**
Titulaire : **Melere, Gilles**
**66 Bis Rue Royale**
**F-74000 Annecy (FR)**

Titulaire : **Millon, Joseph**
**800 Rue Napoléon 1er**
**F-73490 La Ravoire (FR)**
Titulaire : **Fayard, Jean-Philippe**
**Le Rochain**
**F-42170 St. Just sur Loire (FR)**

(72) Inventeur : **Bascoulergue, Gérard**
**45 Bd. Daloz**
**F-62520 Le Touquet Paris Plage (FR)**
Inventeur : **Basso, Maurice**
**57 Avenue Foch**
**F-43000 Le Puy en Velay (FR)**
Inventeur : **Debiesse, Jean-Louis**
**Chemin de Bel Air**
**F-38200 Vienne (FR)**
Inventeur : **Eyraud, Guy**
**Le Guillonnier - Ville sous anjou**
**F-38150 Roussillon (FR)**
Inventeur : **Melere, Gilles**
**66 Bis rue Royale**
**F-74000 Annecy (FR)**
Inventeur : **Millon, Joseph**
**800 Rue Napoléon Ier**
**F-73490 La Ravoire (FR)**
Inventeur : **Fayard, Jean-Philippe**
**Le Rochain**
**F-42170 Saint Just sur Loire (FR)**
Inventeur : **Noyer, Daniel**
**Rue du Vieux Chateau**
**F-38300 Maubec (FR)**
Inventeur : **Collomb, Jean**
**L'Olagnier**
**F-26800 Porte Les Valence (FR)**

(74) Mandataire : **Laurent, Michel et al**
**Cabinet LAURENT et CHARRAS, 20, rue Louis**
**Chirpaz B.P. 32**
**F-69131 Ecully Cedex (FR)**

## Description

L'invention concerne une tige pour prothèse totale de hanche. Selon le préambule de la revendication 1. Une tige de ce type et décrit dans EP-A-0 257-222.

Comme on le sait, une prothèse de hanche comprend essentiellement trois parties, à savoir respectivement :
– une tige fémorale, dénommée également "tige", destinée à être insérée dans le fémur ;
– une cupule cotyloïdienne, dénommée également "cupule", destinée à être engagée dans la cavité cotyloïdienne de la hanche considérée ;
– un col prothétique, coiffé d'une sphère, destiné à relier la tige et la cupule pour assurer ainsi l'articulation proprement dite.

La mise en place de la tige dans le fémur s'effectue essentiellement par deux techniques.

Dans la première, la tige est cimentée. Bien que utilisée depuis longtemps, cette technique présente toutefois des inconvénients per- et post-opératoires susceptibles d'entraîner des risques d'infection. Elle présente également en cours de portée des risques de descellements et enfin des difficultés lors des reprises qui peuvent fragiliser l'os.

Dans la seconde technique plus récente, dénommée "à fixation primaire", la tige est fixée en force dans l'os, notamment par vissage (voir par exemple EP-A-0190981). Cette technique présente toutefois l'inconvénient de provoquer des douleurs post-opératoires résultant semble-t-il des pics de contrainte formés au niveau du pas de vis.

On connait depuis déjà longtemps une tige fémorale de prothèse totale de hanche à fixation primaire, qui présente un profil longitudinal effilé depuis la tête jusqu'à la pointe et une section générale trapézoïdale dans laquelle :
– d'une part, la courbure antérieure de la tige, vue dans un plan longitudinal, coïncide avec la courbure du cintre obturateur du fémur par rapport au cotyle pour faciliter la standardisation ;
– et d'autre part, la portion proximale trochantéro-diaphysaire de la tête de la tige est évasée dans le plan antéropostérieur.

Comme on le sait, le "cintre obturateur du fémur" est la ligne radiologique idéale qui s'étend du bassin jusqu'au fémur et qui correspond à la partie supérieure du trou obturateur en se continuant par le bord inférieur du col du fémur, puis par le bord médial de la métaphyse fémorale proximale.

Pour améliorer la stabilité primaire de cette tige, on a alors suggéré de ménager sur chacune des deux faces latérales, notamment dans la zone proximale, au moins une rainure et de préférence une pluralité de rainures longitudinales parallèles. Toutefois, comme on le sait, dans le temps, la cavité osseuse dans laquelle est insérée la tige, a tendance à se tasser et à s'élargir. Il s'ensuit que la tige a également tendance à descendre dans cette cavité, donc à bouger. Cette instabilité secondaire oblige parfois à une nouvelle opération.

L'invention pallie ces inconvénients. Elle se rapporte à une tige de prothèse de hanche qui soit bien adaptée à la forme de la hanche à renforcer, dans laquelle on diminue notablement les pics de contrainte, et qui présente une excellente stabilité secondaire, même dans le temps.

Le problème est résolu par les caractéristiques de la seconde partie de la revendication 1.

Avantageusement, ces fentes caractéristiques longitudinales ont une forme générale d'ouies de poisson, de manière à assurer une certaine élasticité et par là, une meilleure expansion après la mise en place et ainsi d'obtenir une bonne stabilité secondaire, ce que l'on ne savait faire jusqu'alors.

La forme générale de la tige de l'invention assure comme déjà dit une bonne stabilité primaire. La caractéristique originale des fentes longitudinales en forme d'ouies assure une excellente stabilité secondaire dans le temps. Ainsi, bien que la cavité osseuse a tendance à s'agrandir, donc de permettre à la tige de descendre, les fentes en forme d'ouies, qui ont été resserrées lors de l'insertion, donnent de l'élasticité à l'ensemble, ce qui assure un meilleur placage contre les parois osseuses. Ainsi, ces ouies peuvent s'opposer en coopérant avec les rainures, à ce mouvement de glissement.

Avantageusement, en pratique :
– la surface de la portion distale de la pointe de la tige est lisse, ce qui évite des contraintes en bout de tige ;
– cette tige fémorale est en alliage de titane, notamment du type TA6V et la portion proximale est sablée de manière à présenter un état de surface formé d'une pluralité de micro-cavités de cinquante à cent, de préférence de l'ordre de quatre-vingt, micromètres, de manière à assurer une bonne stabilité et une bonne fixation sans ciment ;
– l'angle de l'axe longitudinal de la tige par rapport à l'axe du col prothétique (angle cervico-diaphysaire) est voisin de 138°.

La manière dont l'invention peut être réalisée et les avantages qui en découlent ressortiront mieux de l'exemple de réalisation qui suit, donné à titre indicatif et non limitatif, à l'appui des figures annexées.

La figure 1 est une représentation vue de face d'une tige de prothèse totale de hanche à fixation primaire conforme à l'invention.

La figure 2 est une représentation en coupe selon l'axe AA de la figure 1.

La figure 3 est une représentation vue postérieure de la tige conforme à l'invention.

Cette tige désignée par la référence générale (1) en alliage métallique, notamment en alliage de titane spécialement du type TA6V est effilée de la tête (2) jusqu'à la pointe (3). De manière connue, la tête (2) est reliée de manière continue au col prothétique (4) qui se termine par un emmanchement tronconique (5) sur lequel vient se loger la sphère d'articulation (6) dans la cupule non représentée et dont l'angle au sommet est voisin de 5°.

La surface extérieure de la portion distale (7) de la pointe de la tige est lisse. Cette portion lisse caractéristique (7) s'étend sur environ le tiers de la tige.

La courbure antérieure de la tige désignée par la référence (8), vue dans un plan longitudinal, coïncide avec la courbure du cintre obturateur du fémur par rapport au cotyle et est voisine de 138°C (angle alpha).

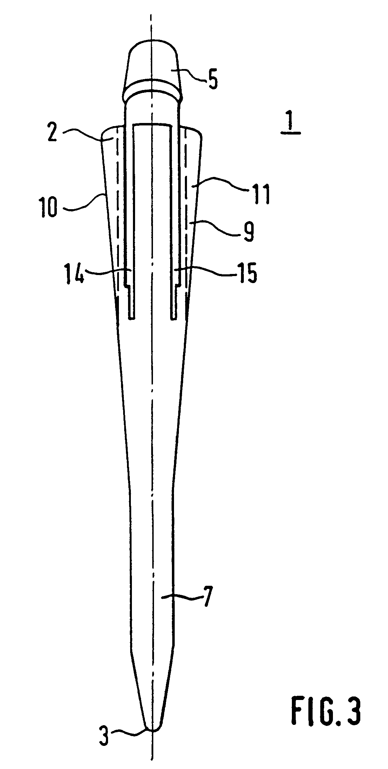

De manière connue, la portion proximale trochantéro-diaphysaire (9) de la tête de la tige (2) d'une part, comme on le voit sur la figure 3 est évasée dans le plan antéro-postérieur depuis le raccordement de la portion lisse (7) jusqu'à la pointe (2) et d'autre part, présente sur ses deux faces principales latérales (10,11) une pluralité de rainrues parallèles (12) et (13) longitudi nales. Ces rainures de profondeur et de largeur égale, par exemple de l'ordre de deux millimètres, sont de longueur variable de la face postérieure jusqu'à la face antérieure.

Cette portion proximale (9) trochantéro-diaphysaire est avantageusement sablée ou revêtue d'un dépôt de plasma ou poreux ou grenaillé, de manière à avoir un état de surface microporeux facilitant la stabilité secondaire, et la reprise de l'os.

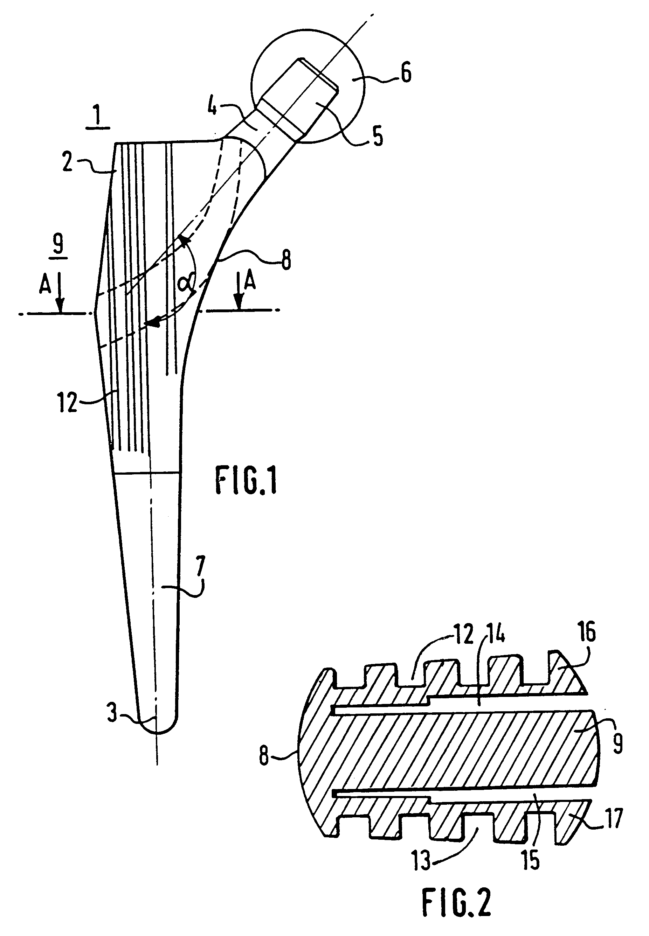

Selon la caractéristique de l'invention (voir figures 2 et 3), la portion trochantéro-diaphysaire (9) présente deux fentes longitudinales parallèles, respectivement (14) et (15) symétriques, en forme d'ouïe de poisson, ouvertes depuis la face antéropostérieure (9) jusqu'à la face antérieure (8), de manière à assurer une meilleure expansion et de donner de l'élasticité lors de la mise en place.

Lors de la mise en place, les joues (16,17) sont comprimées de manière à resserrer les fentes (14,15). Lorsque la tige est en place, les joues sont ensuite relâchées puis viennent alors se bloquer contre les corticales, ce qui assure un remplissage optimal du canal médullaire et une excellente stabilité secondaire.

Comme on le voit sur la figure 2, la section de la tige est essentiellement trapézoïdale dégressive.

Dans une forme de réalisation pratique, les fentes caractéristiques (14) et (15) sont usinées par fraisage. La largeur de ces fentes varie de 1 à 2 mm, la profondeur de 49 à 63 mm et la forme d'ouie est fraisée à la scie selon un rayon de 100 mm.

La tige selon l'invention présente de nombreux avantages par rapport à celles exploitées jusqu'alors. On peut citer sa facilité de mise en place, l'absence de pics de contrainte, et une forme anatomique parfaitement adaptée et une excellente stabilité primaire et surtout secondaire, ce qui l'on ne savait obtenir commodément jusqu'alors. Elle convient donc parfaitement comme tige de prothèse de hanche à fixation primaire.

## Revendications

1. Tige fémorale (1) de prothèse totale de hanche à fixation primaire, qui présente un profil longitudinal effilé depuis la tête (2) jusqu'à la pointe (3), et une section générale trapézoïdale, et dans laquelle :
   – d'une part, la courbure antérieure (8) de la tige (1), vue dans un plan longitudinal, coïncide avec la courbure du cintre obturateur du fémur par rapport au cotyle ;
   – et d'autre part, la portion proximale trochantérodiaphysaire (9) de la tête (2) de la tige (1) :
      . est évasée dans le plan antéropostérieur,
      . et présente sur ses deux faces principales latérales (10,11) des rainures longitudinales (12,13),
   – et dans laquelle, la tige (1) présente des fentes longitudinales symétriques ;
caractérisée en ce que la portion trochantéro-diaphysaire (9) présente deux fentes longitudinales (14,15) en forme générale d'ouies de poisson, parallèles, symétriques, ouvertes depuis la face antéropostérieure (9) jusqu'à la face antérieure (8).

2. Tige fémorale selon la revendication 1, caractérisée :
   – en ce que la surface de la portion distale (7) de la pointe (3) de la tige (1) est lisse ;
   – en ce que les rainures longitudinales (12,13) sont parallèles à l'axe de la tige, et ont une longueur variable qui diminue de la face antéropostérieure (9) à la face antérieure (8) ;
   – et en ce que l'angle de l'axe longitudinal de la tige (1) par rapport à l'axe du col prothétique (4) est voisin de 138°.

## Patentansprüche

1. Oberschenkelschaft (1) einer Hüfttotalprothese zur Primärfixation, der ein längliches Profil aufweist, der sich vom Kopf (2) zur Spitze (3) hin verjüngt und einen trapezförmigen Hauptabschnitt aufweist, wobei
   – einerseits, die anteriore Krümmung (8) des Schaftes (1), in einer längsverlaufenden Ebene gesehen, mit der Krümmung des Schlußbogens des Oberschenkelknochens zur Gelenkpfanne hin zusammenfält ;
   – und andererseits das proximale Trochantermittelstück (9) des Kopfes (2) des Schaftes (1)
      . in der antero-posterioren Ebene erweitert ist,

. und auf seinen beiden Hauptseitenflächen (10, 11) längsverlaufende Nuten (12, 13) aufweist,

– und wobei der Schaft (1) symmetrische Längsspalte aufweist,

dadurch gekennzeichnet, daß das Trochantermittelstück (9), von der antero-posterioren Seite (9) bis zur anterioren Seite (8), zwei parallele, symmetrische, offene Längsspalte (14, 15) in der allgemeinen Form von Fischkiemen aufweist.

2. Oberschenkelschaft nach Anspruch 1, dadurch gekennzeichnet,

– daß die Oberfläche des distalen Abschnittes (7) der Spitze (3) des Schaftes (1) glatt ist;

– daß die längsverlaufenden Nuten (12, 13) parallel zur Schaftachse sind, und verschiedene Längen aufweisen, die von der antero-posterioren Seite (9) zur anterioren Seite (8) abnimmt;

– und daß der Winkel zwischen der Längsachse des Schaftes (1) und der Längsachse des Prothesenhalses (4) etwa 138° beträft.

## Claims

1. Femoral stem (1) for a total hip prosthesis with primary fixation, which has a longitudinal profile tapering from the head (2) to the point (3), and a trapezoidal general cross-section, and in which :

– on the one hand, the anterior curvature (8) of the stem (1), viewed in a longitudinal plane, coincides with the curvature of the obturator arch of the femur relative to the acetabulum;

– and, on the other hand, the proximal trochanterodiaphyseal portion (9) of the head (2) of the stem (1) :

. is widened in the anteroposterior plane,

. and has on its two main lateral faces (10, 11) longitudinal grooves (12, 13),

– and in which the stem (1) has symmetrical longitudinal slots;

characterised in that the trochantero-diaphyseal portion (9) has two longitudinal slots (14, 15) in the general shape of fish gills, which are parallel, symmetrical and open from the anteroposterior face (9) to the anterior face (8).

2. Femoral stem according to Claim 1, characterised:

– in that the surface of the distal portion (7) of the point (3) of the stem (1) is smooth;

– in that the longitudinal grooves (12, 13) are parallel to the axis of the stem and have a variable length which decreases from the anteroposterior face (9) to the anterior face (8);

– and in that the angle of the longitudinal axis of the stem (1) relative to the axis of the prosthetic neck (4) is in the region of 138°.

FIG.1

FIG.2

FIG.3